# EUROPEAN PATENT APPLICATION

(11) **EP 4 790 718 A1**
(43) Date of publication of application: **12.08.2026**
(21) Application number: 25157036.2
(22) Date of filing: 11.02.2025
(51) Int. Cl.: G16H 20/10, G16H 70/40, G16H 80/00

(54) **METHOD FOR ESTABLISHING A DOSAGE REGIMEN, COMPUTER PROGRAM PRODUCT AND MEDICAMENT**

(71) Applicant: medigital GmbH, 58638 Iserlohn (DE)
(72) Inventor: Preiss, Katja, Iserlohn (DE); Lambrecht, Felix, Iserlohn (DE); Dunbar, Allan, Iserlohn (DE)
(74) Representative: Hamm&Wittkopp Patentanwälte PartmbB

(57) **Abstract**

The present invention concerns a method for establishing a dosage regimen for a medicament to be administered to a patient suffering from attention deficit hyperactivity disorder (ADHD) or depression. The inventive method comprises a titration phase with successive steps in which data is obtained and transferred between a first electronic device and a second electronic device. The present invention also concerns a computer program product comprising instructions which, when the program is executed by a computer, cause the computer to carry out the steps of the method and, a medicament for use in a method of treating ADHD or depression.

## Description

The present invention concerns a method for establishing a dosage regimen for a medicament to be administered to a patient suffering from attention deficit hyperactivity disorder (ADHD) or depression. The inventive method comprises a titration phase with successive steps in which data is obtained and transferred between a first electronic device and a second electronic device. Furthermore, the present invention concerns a computer program product comprising instructions which, when the program is executed by a computer, cause the computer to carry out the steps of the method and moreover a medicament for use in a method of treating ADHD or for use in a method of treating depression.

### Prior Art

Attention Deficit Hyperactivity Disorder (ADHD) is one of the most common neurodevelopmental disorders in children and often continues into adulthood. National survey data from 2016 revealed that 9.4% of US children received an ADHD diagnosis at some point and that 8.4% currently had ADHD. It is recognized as a lifelong disorder and remains one of the most extensively studied and yet highly controversial conditions. The characteristics of ADHD are described as a neurodevelopmental disorder resulting in patterns of restlessness, inattention, impulsivity, easy arousability, and hyperactivity, which stem from underlying differences in brain structure and function.

In the US, healthcare professionals (HCPs) primarily use the diagnostic criteria outlined in the American Psychiatric Association's Diagnostic and Statistical Manual of Mental Disorders, Fifth Edition (DSM-5) to diagnose ADHD. In addition to these diagnostic criteria, clinical practice guidelines for ADHD evaluation and treatment are provided by organizations such as the American Academy of Pediatrics (AAP) and the American Academy of Child and Adolescent Psychiatry (AACAP). These guidelines recommend comprehensive assessments that integrate DSM-5 criteria with clinical judgment, patient history, and symptom monitoring across multiple settings. Internationally, a variety of guideline systems exist, many of which incorporate either the DSM-5 or the World Health Organization's International Classification of Diseases (ICD) criteria, or both. These criteria and associated guidelines aim to standardize and optimize the diagnosis and management of ADHD across different healthcare systems, although specific approaches and recommendations may vary by region. HCPs are typically not obligated to follow guidelines but must use ICD and DSM criteria for reimbursment.

This diagnostic standard helps ensure that people are appropriately diagnosed and treated for ADHD. Although the diagnostic criteria for ADHD have evolved over time, the assessment and tools for evaluation have remained essentially the same. ADHD remains a largely clinical diagnosis. Current recommendations for diagnostic evaluation of possible ADHD in children include a comprehensive history taking of prenatal, perinatal, and family history; school performance; environmental factors; psychopathological findings and a detailed physical examination and psychopathological findings. During the physical examination, particular attention should be paid to vital signs (cardiovascular, skin, thyroid, and neurologic systems, including assessment of motor coordination), and a mental health assessment used to probe for comorbid conditions should be performed.

So far, stimulant medications and behavioural interventions have been the mainstay for the treatment of patients with ADHD. Evidence-based psychosocial interventions, such as parental behavioural training, have demonstrated significant efficacy, particularly for children and as part of a comprehensive treatment plan. These interventions help parents and caregivers develop strategies to manage their child's symptoms effectively, fostering improvements in behaviour and family dynamics. Other interventions, including dietary modifications, herbal supplements, EEG biofeedback (neurofeedback), and cognitive behaviour therapy, have shown mixed or limited empirical support for their efficacy. While some of these approaches may offer benefits for specific subgroups of patients, they are not currently considered primary treatments and require further rigorous investigation to establish their effectiveness. Because ADHD continues into adulthood for many individuals, both patients with persistent symptoms and those whose ADHD symptoms have resolved are at risk for emergence of other psychiatric disorders and adverse outcomes as they approach their adult years.

Depression encompasses a broad range of mental health issues, characterized primarily by the absence of positive affect (a diminished interest and enjoyment in everyday activities), persistent low mood, and a spectrum of emotional, cognitive, physical, and behavioral symptoms. It manifests differently across individuals and can present with a wide variety of symptoms, including prolonged feelings of unhappiness and hopelessness, loss of interest in previously enjoyable activities, and frequent tearfulness. Many individuals with depression also experience anxiety. Physical symptoms may accompany emotional and mental challenges, such as constant fatigue, poor sleep, loss of appetite, decreased libido, and various aches or pains.

The severity of depression varies widely. In mild cases, individuals may feel persistently low in spirit, while severe depression can lead to feelings of despair and suicidal ideation. It is important to differentiate between temporary low mood, which often resolves on its own during challenging times, and clinical depression, which requires attention and treatment.

Depression is often treated using a combination of therapeutic interventions, including medication, psychotherapy, and lifestyle changes. Antidepressants are commonly prescribed to alleviate symptoms and restore balance to brain chemistry. These medications fall into several categories, including:
- selective serotonin reuptake inhibitors (SSRIs) such as citalopram, escitalopram, fluoxetine, fluvoxamine, paroxetine, sertraline,
- serotonin-norepinephrine reuptake inhibitors (SNRIs) like desvenlafaxine, duloxetine, levomilnacipran, milnacipran, venlafaxine,
- serotonin-norepinephrine-dopamine reuptake inhibitors (SNDRIs) such as toludesvenlafaxine, nefazodone,
- serotonin modulators and stimulators (SMSs) like vilazodone, vortioxetine,
- serotonin antagonist and reuptake inhibitors (SARIs) like nefazodone, trazodone,
- norepinephrine reuptake inhibitors (NRIs) like reboxetine, teniloxazine, viloxazine,
- tricyclic antidepressants (TCAs) such as amitriptyline, amitriptylinoxide, amoxapine, clomipramine, desipramine, dibenzepin, dimetacrine, dosulepin, doxepin, imipramine, lofepramine, melitracen, nitroxazepine, nortriptyline, noxiptiline, pipofezine, protriptyline, trimipramine.

Changes to medication doses to achieve the best clinical response is known as drug titration. The scientific principles of drug titration are to give the patient adequate and effective treatment, at the lowest dose possible, with the aim of minimising unnecessary medicament use and side effects. Drug titration involves the interplay of scientific titration principles with the clinical expertise of the healthcare professionals or healthcare provider (HCP), and an individualised, patient-centred partnership between the provider and the patient to review the delicate balance of perceived benefits and risks from both perspectives. Drug titration may occur as up-, down-, or cross-titration depending on whether the goal is to reach or maintain a therapeutic outcome, decrease the risk of adverse effects, or prevent withdrawal/discontinuation syndromes or recurrence of symptoms.

Drug titration is used routinely in general medicine and psychiatry to ensure optimal clinical effect, while minimising the risk of adverse effects. Such a titration is common for almost all psychotropic medications and is crucial, particularly when considering clinical dose-response relationships with high interindividual variation, such as the use of stimulants for treatment of ADHD or the treatment of depression with antidepressants. Various factors can influence the need for dose optimization including genetic variability, patient weight, age, drug-induced tolerance, interactions with other medications or medical conditions, and work or school conditions. The length of time required and the method used to optimize dosage can vary extensively between pharmacological treatments. Titration for antidepressants is similar to that for ADHD stimulants; however, the primary difference lies in the time required for titration. While the effects of stimulants can often be assessed within hours, antidepressants typically take several weeks to show a measurable response, making the titration process significantly longer.

Methylphenidate (MPH) is a central nervous system stimulant, that is commonly used for pharmacological treatment of adults and children with ADHD. Current practice guidelines recommend optimising methylphenidate dosage to individual patient needs. For example, the *National Institute for Health and Care Excellence* (NICE) Guidelines for children and adolecents recommended an initial treatment with methylphenidate with low doses of immediate-release (5 mg one or two times, daily) or modified-release preparations (10mg once daily in the morning). The dose needs to be titrated against symptoms and side effects over approximately 4-6 weeks until a dose optimisation is achieved. The maximum dosage of methylphenidate in children and adolescents is up to 60 mg/day.

ADHD pharmacological treatment options extend beyond methylphenidate, encompassing a broader range of stimulant and non-stimulant medications tailored to individual patient needs. For example, stimulant medications include amphetamine-based drugs like adderall. Additionally, non-stimulant medications like atomoxetine, a selective norepinephrine reuptake inhibitor, and alpha-2 adrenergic agonists such as guanfacine, are available for patients who have contrindications, do not respond well to stimulants or experience significant side effects. Off-label medications, including bupropion and antidepressants, may also be considered in cases with specific comorbidities, further expanding the range of therapeutic possibilities. These diverse options are all comprised by the present invention and clearly highlight the importance of a tailored approach to ADHD treatment, optimizing outcomes through personalized medication selection and dosage adjustments.

The primary goal of ADHD treatment is to manage symptoms such as inattention, hyperactivity, impulsivity, and emotional dysregulation while minimizing the impact on the patient's daily functioning and quality of life. The efficacy of medication is typically evaluated through subj ective measures, which capture improvements in these symptoms and overall behavioural and cognitive performance.

Key symptoms monitored during ADHD treatment include the ability to sustain attention on tasks, follow instructions, and regulate impulses, as well as reductions in hyperactive behaviour. Behavioural and emotional changes, such as improved peer interactions, reduced frustration, and better adaptability to routine, are also critical markers of treatment success. These symptoms are evaluated through caregiver, teacher, and patient feedback, often using structured questionnaires. Teacher feedback is particularly valuable as it provides insight into the patient's performance in a structured learning environment. These add to the clinical impression of the HCP.

While stimulant medications, such as methylphenidate and amphetamines, are widely regarded as the cornerstone of ADHD treatment, their use is not without risks. Common side effects include decreased appetite, insomnia, headache, and stomach-ache, which can often be managed with dose adjustments or changes in administration timing. However, more serious adverse effects, such as cardiovascular issues (e.g., increased blood pressure and heart rate), psychiatric symptoms (e.g., increased anxiety, irritability, or exacerbation of pre-existing mood disorders), and rare but serious concerns about growth suppression in children, highlight the need for cautious administration and monitoring.

Safety concerns specific to ADHD medication extend to the risk of misuse and dependency, particularly with stimulant drugs, which are classified as controlled substances in many regions. Proper titration and adherence to prescribed doses are essential to minimize these risks, especially in adolescent and young adult populations.

Non-stimulant options, such as atomoxetine or guanfacine, present alternative treatments but come with their own safety profiles, including risks of sedation, gastrointestinal discomfort, and potential liver toxicity. Balancing the therapeutic benefits with these safety considerations requires a patient-centred approach that considers the unique physiological and psychological characteristics of each patient.

To ensure comprehensive safety monitoring, HCPs track a range of side effects during titration. Cardiovascular parameters, such as blood pressure, heart rate and electrocardiogram, are routinely measured to detect potential stimulant-induced cardiovascular effects. Blood tests may be conducted periodically to monitor liver function, especially in patients on non-stimulant medications like atomoxetine, which carries a risk of liver toxicity. Headaches and gastrointestinal symptoms are monitored through patient or caregiver feedback, often facilitated by structured questionnaires. Behavioural and emotional side effects, such as increased anxiety or mood swings, are assessed via patient-reported outcomes or input from teachers and caregivers.

These factors underscore the importance of careful titration and ongoing monitoring during ADHD and depression treatment to achieve the optimal balance between efficacy and safety, and there are several known problems with titration:
While the majority of international guidelines recommend titration for medication management in ADHD (a similar approach is often advised for certain antidepressants in the treatment of depression), they often lack clear, standardized methodologies for implementation. This absence of explicit guidance results in significant variation in clinical practices, leading to inconsistencies in dose adjustments, extended titration periods, and suboptimal treatment outcomes. Furthermore, without a structured framework, healthcare providers face challenges in achieving patient adherence, accurately monitoring symptoms and side effects, and tailoring treatment to individual patient needs.

These challenges are compounded by the inherently time-consuming and complex nature of titration. Family resistance to medication, particularly higher doses of psychotropic drugs such as methylphenidate, further complicates the process. As a result, drug titration is sometimes skipped entirely, and in many cases, only a basic, minimal dose of medication is prescribed. This approach foregoes the iterative cycles necessary to optimize patient response to a specific drug.

Adherence during the titration period is another critical issue. Consistently taking medication as prescribed is essential for accurate dose adjustment, yet adherence is often unreliable. Caregivers may inaccurately report medication usage, adding further complexity to the titration process. Additionally, patients or caregivers may experience "reporting fatigue," leading to incomplete or inconsistent feedback, which undermines the accuracy of symptom tracking.

Moreover, the titration process rarely accounts for the patient's unique daily routine or fluctuating symptoms due to the lack of real-time feedback mechanisms. This limitation prevents precise timing of medication administration, which is essential for optimal symptom control throughout the day. The absence of intraday data collection tools hinders the ability to fine-tune dose regimens based on the patient's varying activity levels and cognitive demands.

Safety concerns present another significant problem. Without continuous monitoring, potentially serious side effects - such as cardiovascular or behavioral issues-may go unnoticed, delaying necessary adjustments. Current methods also fail to systematically capture or analyze physiological data, such as heart rate, blood pressure, or sleep patterns, which are critical for ensuring the safe and effective use of ADHD medications.

The lack of integration between subjective feedback, objective metrics, and electronic health records further complicates the titration process. Existing tools do not seamlessly combine symptom tracking, side-effect monitoring, and dose regimen data, leaving healthcare providers without a comprehensive view of patient progress. This fragmentation reduces the efficiency of clinical decision-making and increases the risk of medication errors.

Additionally, many current titration approaches rely heavily on in-person consultations, which place a significant logistical and financial burden on patients and their families. This barrier can lead to missed appointments, delayed dose adjustments, and reduced access to optimal care, especially for patients in remote or underserved areas.

Several emerging technologies have been developed or researched to support and streamline the drug titration process, which supports HCPs to achieve an optimal dosage regimen more efficiently. For example, software systems for standardized feedback and improved communication facilitate a regular and structured input from parents or patients. Such software systems shall reduce the administrative burden and improve the communication between parents/patients with HCPs. Also, specific pharmacokinetic optimization tools are known from the prior art that use drug-specific pharmacokinetic profiles to help optimize release duration and timing. Such pharmacokinetic optimization tools shall ensure a more accurate and sustained symptom management. Objective testing tools like continuous performance tests (CPT) or eye tracking have been developed to provide objective measures of symptom changes in response to medication. Objective testing tools shall allow a more quantitative basis for drug titration rather than subjective observations. Wearable devices for physiological monitoring can measure physiological parameters (e.g., heart rate variability, activity levels) that change in response to medication. These wearables may offer real time monitoring and enable additional insights into medication effects, such as whether medications were taken or not, as well as average or temporal impact of a particular medication for an individual. Recent research has also explored the use of machine learning to predict individual responses to ADHD medications aiming to enhance the precision of titration. Studies indicate that integrating machine learning with comprehensive patient data can potentially predict medication responses in ADHD. Similar advancements may also benefit the management of depression, where titration processes for antidepressants could be optimized by leveraging pharmacokinetic tools, wearable devices, and predictive algorithms to achieve more personalized and effective treatments.

Despite the potential of these emerging technologies for ADHD drug titration, several significant barriers limit their adoption in clinical practice, for example:
None of these methods are sufficient on their own. While each provides valuable insights for the titration process, they do not offer a comprehensive picture in isolation. Currently, no solutions integrate multiple approaches effectively. Further, none of these technologies are covered by health insurance, resulting in high out-of-pocket costs for healthcare providers (HCPs) and patients.

Most of the available software systems for standardized feedback and improved communication systems are general-purpose questionnaire tools and do not focus specifically on titration. They also fail to support guideline compliance or safety considerations. These systems do not capture, store or communicate specific dose regimens, which limits their utility and potential. Without this feature, critical functions like enhanced analytics, electronic prescriptions, decision support algorithms or real-world data collection for evidence are not feasible which inhibits many advances. Furthermore, this is a critical safety consideration. Some systems offer HCP-carer/parent messaging but lack robust features for dose regimen communication. The integration with existing electronic health record (EHR) or patient management systems is often absent, creating inefficiencies and challenges in busy clinical environments.

None of the systems are developed as medical devices, limiting their functionality, regulatory approval, and potential adoption. This limitation is also a critical barrier to obtaining health insurance coverage. They do not integrate with other solutions like wearables, reducing their potential as comprehensive tools.

Pharmacokinetic optimization tools lack robust clinical evidence to support their utility. These tools provide only high-level insights. Pharmacokinetic curves represent population averages and fail to address the significant individual variability in temporal drug response. For example, while they may indicate whether a medication generally lasts roughly 2 or 8 hours, they do not offer personalized insights.

Objective testing tools, such as continuous performance tests and eye tracking, lack robust clinical evidence to support their utility, and the absence of established population norms limits their interpretability and application. These tools require a substantial time commitment from both patients and healthcare professionals, typically 10-30 minutes per session, and often necessitate additional hardware, which increases complexity and cost. Furthermore, they provide insights that are limited to symptoms without addressing other critical dimensions of titration. Similarly, wearable devices for physiological monitoring suffer from a lack of sufficient clinical evidence to validate their use, and the absence of established population norms further restricts their interpretability. These devices depend on additional hardware, such as smartwatches, which may not be readily available or affordable, and they offer insights confined to physiological impact without addressing other essential aspects of titration. Machine learning models for predicting drug response also face limitations, including insufficient clinical evidence to substantiate their effectiveness and a scarcity of high-quality datasets for robust modeling. Current models rely solely on pre-titration data and fail to adapt dynamically based on ongoing titration outcomes during the process.

Considering these details, an overarching barrier is the lack of comprehensive data, including dose regimen information. For individual patients, such data could enhance titration analytics and enable more automated and streamlined prescription processes. On a broader scale, such multi-patient datasets would facilitate significant advancements in titration solutions. For example, enhanced clinical evidence could bolster healthcare provider (HCP) confidence, drive adoption, and secure support from insurance providers. Additionally, advanced decision-support and predictive algorithms could be developed to optimize titration processes, and population norms could be established to improve the interpretation and applicability of results across diverse patient groups.

Currently, clinical trials remain the primary means of generating such data. However, these trials are resource-intensive, inefficient, and limited in scope, often resulting in small, incomplete datasets that fail to capture the variability observed in real-world settings.

There remains a need for improved outcomes in the treatment of ADHD and depression. In clinical practice, wide variations in titration schedules exist due to a lack of evidence and consensus on titration approaches that achieve an optimal benefit-harm profile. Further, drug titration can be challenging for patients to follow, resulting in suboptimal adherence and may require increased healthcare-related visits and coordination of care amongst providers.

### Object of the invention

One object of the present invention was to provide a method specifically designed to address the challenges of medication titration in the treatment of ADHD and depression, with a focus on supporting guideline compliance and capturing, storing and communicating comprehensive data including but not limited to symptoms, side effects and dose regimen data. This method improves efficiency of the titration process and offers more complete data used for titration analytics and decision making as well as safety considerations. Preferably, the method incorporates medical device compliance, ensuring functionality and alignment with regulatory standards to enhance its adoption and effectiveness.

The proposed method facilitates the integration of multiple approaches, moving beyond subjective observations to leverage insights from emerging technologies. By capturing all relevant data, including dose regime, the method generates more comprehensive generation of real-world data. These features not only enhance clinical decision-making on an individual basis but also support and amplify the utility of other emerging solutions, fostering a more cohesive and effective ecosystem for ADHD and depression treatment with improved adoption.

This and other benefits are achieved by the subject matter of the independent claims. Preferred embodiments are part of the dependent claims or further explained below.

### Description of the invention

Established clinical approaches for establishing dosage regimens for the treatment of patients suffering from ADHD or depression often fall short due to their time-consuming nature, lack of convenience, and missing adherence of the patients, particularly during the drug titration period. To address these challenges, a novel method for establishing a dosage regimen for a medicament to be administered to a patient suffering from ADHD or depression is proposed, making it perfect for discreet use in any setting.

The present invention concerns a method for establishing a dosage regimen for a medicament to be administered to a patient suffering from ADHD or depression comprising a titration phase (t), wherein the titration phase comprises the following steps:
(11) providing baseline data to a first electronic device, wherein the first electronic device is a processing unit, wherein the baseline data includes basic symptoms and weight of the patient to be treated,
(t2) initiating the titration by defining on the first electronic device at least: an initial medicament, an initial dose, and an initial frequency based on the baseline data to obtain initial titration data,
(t3) transferring the initial titration data from the first electronic device to a second electronic device,
(t4) accessing the initial titration data on the second electronic device,
(t5) providing initial symptoms and/or initial side effects to the second electronic device to obtain initial symptoms data,
(t6) transferring the initial symptoms data from the second electronic device to the first electronic device,
(t7) adding the initial symptoms data to the initial titration data to obtain initial symptoms evaluation data.

Moreover, the present invention concerns a computer program product comprising instructions which, when the program is executed by a computer, cause the computer to carry out the steps of the method according to the invention. The computer program product may operate across the first and second electronic devices through a communication infrastructure designed to ensure seamless and secure data synchronization. This infrastructure may include various technologies, such as cloud-based systems for centralized data processing, peer-to-peer communication for direct device-to-device interaction, or local data storage and retrieval systems. The infrastructure ensures the integrity and confidentiality of transmitted data, complying with relevant data protection standards. Further comprised by the present invention is a medicament for use in a method of treating ADHD or depression, wherein the dosage regimen is established by the inventive method or by the computer program product according to the invention.

The effects of medication for ADHD and depression are highly depending on the patient. Different parameters usually require optimisation and monitoring during the treatment. Parameters that influence the efficacy of ADHD treatment and depression treatment and need to be monitored by a healthcare provider (HCP) include for example the dose size which is a narrow therapeutic window that varies by patient. A too low dose may be ineffective, while a too high dose may cause side effects. Further, the release duration might be crucial. For instance, different life styles suit different durations of the medicament. Also, standard medications used in ADHD treatment or depression treatment come in immediate release, extended release, or sustained release. Moreover, the timing, i.e., when during the day to take medication is important because symptoms generally fluctuate through the day, especially with changes in activities of the person to be treated. Also, pharmacokinetic curves vary between individuals. Of course, the medication type has an influence on the treatment as different patients respond differently on different drug types and not every patient reacts on every drug type. Common drug types used in the treatment of ADHD include stimulant drugs (such as methylphenidate or methylphenidate-based drugs and amphetamine-based drugs) and non-stimulant drugs. Generally, methylphenidate (including pharmaceutical acceptable salts thereof) is the first choice in the treatment of ADHD while non-stimulant drugs are less prominent.

Usually, antidepressants are used in the treatment of depression. These include for example:
- selective serotonin reuptake inhibitors (SSRIs) such as citalopram, escitalopram, fluoxetine, fluvoxamine, paroxetine, sertraline,
- serotonin-norepinephrine reuptake inhibitors (SNRIs) like desvenlafaxine, duloxetine, levomilnacipran, milnacipran, venlafaxine,
- serotonin-norepinephrine-dopamine reuptake inhibitors (SNDRIs) such as toludesvenlafaxine, nefazodone,
- serotonin modulators and stimulators (SMSs) like vilazodone, vortioxetine,
- serotonin antagonist and reuptake inhibitors (SARIs) like nefazodone, trazodone,
- norepinephrine reuptake inhibitors (NRIs) like reboxetine, teniloxazine, viloxazine,
- tricyclic antidepressants (TCAs) such as amitriptyline, amitriptylinoxide, amoxapine, clomipramine, desipramine, dibenzepin, dimetacrine, dosulepin, doxepin, imipramine, lofepramine, melitracen, nitroxazepine, nortriptyline, noxiptiline, pipofezine, protriptyline, trimipramine.

Before the titration begins, in **step t1** baseline data is provided to a first electronic device. This may be conducted through a web-based application or any secure, digital interface. The baseline data includes at least basic symptoms (for example restlessness, inattention, impulsivity, easy arousability, or hyperactivity in the treatment of ADHD and symptoms like persistent sadness, loss of interest or pleasure, fatigue, changes in sleep or appetite, and difficulty concentrating when treating depression) and the weight of the patient to be treated. In another embodiment, the baseline data additionally comprises further parameters such as for example: blood pressure, heart rate, height, sleep patterns, medication history, comorbid conditions (e.g., anxiety, depression, or learning disabilities), prior treatment responses, family medical history, diet and lifestyle factors, and any known allergies or contraindications.

The first electronic device may be operated by an HCP which can be an individual health professional or a health facility organization licensed to provide health care diagnosis and treatment services including medication and medical devices. Preferably, the first electronic device comprises a software which enables the first electronic device to securely transferring data, preferably to electronic patient files/electronic medical-records etc. The first electronic device is a processing unit and can be for example a computer, laptop, mobile phone, tablet computer or similar. The first electronic device does not inherently have a medical purpose and is not necessarily classified as medical devices under most regulatory frameworks unless it is specifically adapted or marketed for medical use. The role of the first electronic device is to provide the necessary computing resources (e.g., processing power, display, and connectivity) for the software to operate. In one embodiment, the first electronic device can serve as a platform to execute medical software (e.g., software as medical device (SaMD)). Software as a Medical Device (SaMD) refers to standalone software designed to perform medical functions, such as diagnosing, monitoring, or managing a medical condition, independently of any specific hardware medical device. The SaMD described in this application provides functionality for titration of medication and operates on general-purpose electronic devices. As long as the first electronic device remains unaltered and is not marketed specifically for medical use, it is not considered as an electronic medical device under frameworks such as the FDA's 21 CFR (Code of Federal Regulations, Title 21), which governs food and drugs in the United States, including medical devices and software, or the EU MDR (Medical Device Regulation, Regulation (EU) 2017/745), which defines and regulates medical devices in the European Union, including software that qualifies as a medical device.

In one embodiment according to the invention, the baseline data is provided by the HCP onto the first electronic device. In another embodiment, the baseline data is provided by the patient to be treated or a carer of the patient to be treated. This may be realised for example via a questionnaire on the second electronic device (web-based or mobile-application) that is completed by the patient/carer and transferred onto the first electronic device afterwards.

In **step t2** the titration is initiated by defining initial titration data that is based on the baseline data provided in step t1. The initial titration data defines at least: an initial medicament, an initial dose, and an initial frequency (how often and point in time). Preferably, the initial titration data comprises parameters for monitoring the progress, including the follow-up frequency for the analysis of possible symptoms of the patient to be treated, as well as the frequency and type of patient or carer-reported feedback required during the titration process to assess the patient's response to the prescribed medication.

The initial titration data is then in **step t3** transferred from the first electronic device to a second electronic device that can be accessed by the patient to be treated or a carer of said patient. The second electronic device may be for example a computer, laptop, mobile phone, tablet computer or similar.

In **step t4,** the initial titration data is accessed on the second electronic device by the patient or the carer of the patient. Based on the accessed initial titration data that includes the initial medicament, the initial dose, and the initial frequency the titration is commenced. In a preferred embodiment, the initial titration data also includes a prescription from the HCP which allows the patient or the carer to obtain the required initial medicament from a pharmacy. Alternatively, the patient or the carer may already have the required medicament or the prescription may be sent from the HCP via mail to the patient/carer. It is preferred that a prescription may be sent electronically from the first electronic device of the HCP to the second electronic device of the patient/carer and the prescribed medicament can be obtained from a pharmacy or online-pharmacy or similar. In yet another embodiment, the HCP can transfer the prescription to the pharmacy which in turn despatches the medicament to the patient or carer.

In **step t5,** occurring initial symptoms and/or initial side-effects are provided to the second electronic device in form of initial symptoms data. This may be conducted for example through measures like a questionnaire or a web-based application that is installed on the second electronic device.

To reduce the burden on carers and patients, streamlined approaches can be employed to make the symptoms data reporting more efficient and manageable. To achieve this, in one embodiment short-form questionnaires may be used. Instead of lengthy assessments, such as the "Fragebogen zur Beurteilung der Behandlung" (FBB), shorter, focused questionnaires like the DAYAS can be used. These concise assessments can still provide valuable information to the HCP while significantly reducing the time required for carers or patients to complete each feedback session.

In another embodiment, the questionnaire gets broken down into smaller, manageable sections. This allows patients or carers to answer questions over multiple days. For instance, one day could focus on symptoms, while another day focuses on side effects. Alternatively, asking just a few questions each day and aggregating results for the HCP review can minimize daily reporting demands while still providing a comprehensive dataset over time.

Other possibilities to ease the procedure of reporting symptoms and/or side effects that are covered by the present invention are
- employing optimized interfaces, such as touch-screen based tree configurations, that can streamline data entry. These interfaces allow patients or carers to quickly navigate through options by selecting pre-set pathways, reducing input time and cognitive load. Research has shown that touch-based decision trees can cut down completion time, making it easier for users to provide feedback accurately and efficiently;
- personalised questions sets for each patient; a personalized questionnaire is an individualized assessment tool designed to capture a specific patient's unique experiences, symptoms, and contexts, providing tailored insights for more effective treatment planning.

Beyond symptom and side effect reporting, collecting additional patient-reported data can provide a more holistic view of medication impact. For instance, the symptoms data may include
- physiological measurements, such as weight, blood pressure, pulse rate, height;
- functional assessments, such as questions on daily activities, eating habbits e.g, reduced sugar intake, household to do list, or cognitive focus; these may help HCPs fine-tune dosage levels, especially when evaluating subtle medication effects on quality of life;
- open-format diary or calendar entries, allow for more nuanced insights, capturing individual experiences and responses not covered in standardized questionnaires;
- feedback from teachers for children or life partners for adults can provide HCPs with a well-rounded view of the child's behaviour, cognitive focus, and social interactions in a structured environment outside the home. Teachers are uniquely positioned to notice changes in attention, impulsivity, and task persistence during different times of the day, as well as responses to various types of tasks (e.g., listening to lectures, working on assignments, group activities);
- objective tests (e.g., Continuous Performance Test - CPT) provide HCPs with quantifiable metrics to assess medication impact. For instance, tests like the CPT can provide data on cognitive function and alertness, helping to track medication effectiveness in relation to mental performance. Additionally, eye-tracking assessments are emerging as valuable objective tools for evaluating ADHD-related impairments. By analyzing gaze patterns, fixation durations, and response times, eye-tracking technology can measure attention deficits, impulsivity, and hyperactivity in real-time. Research also shows the potential of eye tracking to determine if a patient responds to a stimulant or not which could significantly reduce cross-titration timelines. These metrics offer deeper insights into how ADHD impacts visual attention and cognitive processing, further enhancing the ability to assess treatment efficacy and tailor interventions to the individual patient's needs,
- physiological parameters tracked by wearable devices: allows for objective, continuous monitoring of patient health metrics, such as: heart rate, physical activity, or sleep patterns, which can indicate the physiological effects of medication,
- Confirmation of medication used via packaging barcodes or QR code scans.
- HCP notes.

In one embodiment, the initial titration data contains information on the frequency of the symptoms to be reported. In a preferred embodiment, the second electronic device sends out a reminder in case the initial symptoms and/or initial side-effects have not been provided in due course. The reminders might be in the form of a sound played by the second electronic device. Alternatively, such reminders can be in the form of an email or a phone call. In another embodiment, the second electronic device provides reminders for medication adherence and offers access to safety information based on the prescribed medication, dose, and frequency.

In **step t6,** the initial symptoms data obtained in step t5 are subsequently transferred from the second electronic device to the first electronic device. In **step t7,** the initial symptoms data that include initial symptoms and/or initial side effects are added to the initial titration data to give initial symptoms evaluation data.

In one embodiment, if the initial symptoms evaluation data obtained in step t7 require further adjustment, the titration phase is repeated with the following steps:
(t2.1) initiating an adjusted titration by defining on the first electronic device at least one of: an adjusted medicament, an adjusted dose, and an adjusted frequency based on the initial symptoms evaluation data to obtain an adjusted titration data,
(t3.1) transferring the adjusted titration data from the first electronic device to the second electronic device,
(t4.1) accessing the adjusted titration data on the second electronic device,
(t5.1) providing symptoms and/or side effects to the second electronic device to obtain adjusted symptoms data,
(t6.1) transferring the adjusted symptoms data from the second electronic device to the first electronic device,
(t7.1) adding the adjusted symptoms data to the initial symptoms evaluation data to obtain adjusted symptoms evaluation data.

In step **t2.1,** an adjusted titration data is obtained for initiating an adjusted titration. The adjusted titration data differs from the initial titration data of step t2 by at least one of: an adjusted medicament, an adjusted dose, and an adjusted frequency. The adjustment is depending on the initial symptoms evaluation data and may lay in the discretion of the HCP. The adjusted titration data is in **step 3.1** transferred from the first electronic device to the second electronic device and subsequently, in **step t4.1,** accessed on the second electronic device by the patient or the carer of the patient. Based on the accessed adjusted titration data that includes at least one of an adjusted medicament, an adjusted dose, and an adjusted frequency, the titration is commenced.

In one embodiment, the adjusted titration data also includes a prescription from the HCP which allows the patient or the carer to obtain the required adjusted medicament from a pharmacy. Alternatively, the prescription may be sent from the HCP via mail to the patient/carer. It is preferred that a prescription may be sent electronically from the first electronic device of the HCP to the second electronic device of the patient/carer and the prescribed medicament can be obtained from a pharmacy or online-pharmacy or similar. In yet another embodiment, the HCP can transfer the prescription to the pharmacy which in turn despatches the medicament to the patient or carer.

Paper prescriptions involve the physical documentation of medication details provided by the healthcare professional (HCP) to the patient. Despite their simplicity, they are prone to issues such as illegible handwriting, loss, and forgery. Paper prescriptions remain widely used in settings with limited digital infrastructure or for patients who prefer tangible documentation. In some jurisdictions, this remains the only legally accepted prescription method for narcotics and controlled substances, including stimulants used for ADHD, though this may change as digital prescribing systems become more widely adopted.

Electronic prescriptions allow HCPs to transmit medication orders directly to pharmacies or patients via secure digital platforms. Benefits include eliminating handwriting errors, automated drug interaction checks, and seamless integration with electronic health records (EHRs). E-prescriptions expedite the prescription process and ensure accurate documentation, though they require robust cybersecurity measures to protect patient data.

Telemedicine enables HCPs to prescribe medications remotely during virtual consultations. These prescriptions are typically issued electronically, maintaining continuity of care for patients in remote or underserved areas. However, telemedicine prescriptions are subject to jurisdictional regulations, particularly for controlled substances, and require secure platforms for issuance.

Mobile health (mHealth) applications offer patients direct access to prescriptions through digital platforms. Features include digital prescription storage, medication reminders, and integration with wearable devices to track adherence and adjust doses. While convenient, these methods require careful handling of patient data and equitable access for those without internet or smartphone access.

In certain cases, such as follow-ups or chronic condition management, prescriptions may be sent via secure email or encrypted messaging systems. These methods are straightforward and accessible but must comply with data protection laws, such as HIPAA (the U.S. Health Insurance Portability and Accountability Act) or GDPR (the EU's General Data Protection Regulation), to ensure patient confidentiality.

In **step t5.1,** occurring symptoms and/or side-effects are provided to the second electronic device in form of adjusted symptoms data. This may be conducted for example through measures like a questionnaire or a web-based application that is installed on the second electronic device. Like in step t5, additional patient-reported data as part of the adjusted symptoms data may be provided that might give a more holistic view of medication impact.

Similar to step t5, in one embodiment, the adjusted titration data obtained in step 2.1 contains information on the frequency of the symptoms and/or side effects to be reported. In a preferred embodiment, the second electronic device sends out a reminder in case the adjusted symptoms and/or adjusted side-effects have not been provided in due course. The reminders might be in the form of a sound played by the second electronic device. Alternatively, such reminders can be in the form of an email or a phone call. In another embodiment, the second electronic device provides reminders for medication adherence and offers access to safety information based on the prescribed medication, dose, and frequency.

In **step t6.1,** the adjusted symptoms data obtained in step t5.1 are subsequently transferred from the second electronic device to the first electronic device. In **step t7.1,** the adjusted symptoms data that include symptoms and/or side effects are added to the adjusted titration data on the first electronic device to give adjusted symptoms evaluation data.

Based on the initial symptoms evaluation data and/or the adjusted symptoms evaluation data, the treatment is continued with the same medication type, dose, and frequency if the initial symptoms evaluation data and/or the adjusted symptoms evaluation data aligns with expected outcomes.

Alternatively, the dose or frequency may be adjusted, including the issuance of an updated electronic prescription if neccessary, which is securely transferred to the patient or carer through the digital interface, along with revised instructions. Preferably, steps t2.1 to t7.1 are repeated until no further adjustment of the titration is necessary. In one preferred embodiment, after step t7 or, if necessary, after step t7.1, a monitoring phase m is commenced.

In a preferred embodiment, the second electronic device provides regular reminders prompting the patient or carer to enter symptoms and/or side effect data at the designated times. This ensures that the data collection aligns with the HCP's monitoring requirements.

The proposed method can systematically capture and integrate dose regimen data alongside symptom, side effect, etc. tracking. This feature addresses a significant gap in existing software solutions and offers several critical benefits. In a preferred embodiment, the initial symptoms evaluation data and/or the adjusted symptoms evaluation data may be reviewed by the HCP. The initial symptoms evaluation data and/or the adjusted symptoms evaluation data may be displayed in visual formats (e.g., graphs or trend lines) or as a concise summary which makes it easier for the HCP to quickly assess the patient's progress and identify any patterns or concerns during the drug titration.

Dose regimen data might be essential for meaningful analysis of the titration process. By capturing precise details of administered doses, the method allows for a deeper understanding of dose-response relationships. Tracking the connection between dose changes and symptom variations ensures that HCPs can evaluate the effectiveness of specific doses in managing symptoms. Without this data, it becomes challenging to determine whether observed symptom improvements are directly attributable to medication adjustments or influenced by external factors.

Titration may involve incremental adjustments over time. Dose regimen tracking enables temporal mapping of changes in symptoms or side effects relative to dose modifications, providing critical insights into the dynamics of treatment response.

The data may be visualized in line graphs which display symptom severity over time, overlaid with dose changes, to identify patterns and correlations. Alternatively, it may be visualized by heatmaps that illustrate symptom improvement or side effect intensities at different dose levels, enabling quick identification of optimal doses.

Without dose regimen data, the presentation of results might be limited. Only capturing symptoms or side effects creates ambiguity, as these could be influenced by numerous variables unrelated to the medication. Incorporating dose data significantly enhances precision. Variations in symptoms can occur due to factors like stress, sleep, or comorbid conditions. By linking symptoms to specific doses, the method ensures that any associations are contextualized, offering greater confidence in the interpretation of results. Dose regimen tracking enables HCPs to pinpoint the optimal dose for each patient, minimizing unnecessary trial-and-error adjustments.

The results can be visualized by scatter plots that correlate dose levels with specific symptom changes or side effects to highlight trends and anomalies. They might be visualized by bar charts that compare average symptom scores across different dose ranges to identify the most effective dose windows.

The ability to capture dose regimen data sets and combining this data with symptom and side effect tracking, the method provides HCPs with contextualized, precise, and actionable insights necessary to optimize ADHD treatment. Advanced visualizations, such as line graphs, scatter plots, and dynamic dashboards, further enhance the usability of this data by making complex relationships between dose regimens and treatment outcomes readily interpretable.

In one embodiment the method includes disclaimers that clarify that the symptom tracking is intended for feedback purposes only and should not be used for independent treatment decisions by the patient or carer. All collected data is securely transmitted between the first electronic device and the second electronic device, ensuring confidentiality and data integrity in line with healthcare data regulations that are applicable.

The present invention further comprises a computer program product comprising instructions which, when the program is executed by a computer, cause the computer to carry out any of the steps of the inventive method. In one embodiment, the computer program product running on the first electronic device comprises logic-based algorithms that may offer structured, rule-based suggestions to the HCP by interpreting the data collected from the patient or carer. These algorithms may be programmed with specific rules that consider various symptoms, side effects, and other patient-reported metrics to provide consistent, standardized guidance. For example, if certain side effects exceed a predefined threshold, the system could suggest a dose reduction. Alternatively, if side effects are minimal and symptoms are improving, the computer program product might recommend to maintain the current dose. This approach offers straightforward support without requiring extensive data training and can help HCPs to make data-informed decisions more quickly.

In another embodiment, data-driven decision support can leverage machine learning algorithms to analyse patterns from large datasets of historical patient data. Using patient data combined with historical outcomes from multiple patients, machine learning models can provide tailored recommendations based on similar cases. This method allows for predictive suggestions that account for complex relationships in the data, which may not be apparent through simple rule-based logic. Moreover, the adaptation over time as more data is added, can improve the accuracy and relevance of recommendations for future titration adjustments.

Such data-driven support systems can potentially offer more personalized, dynamic insights and can support HCPs to make well-informed decisions based on both individual patient feedback and broader population data trends.

Further comprised by this invention is a medicament for use in a method of treating ADHD or of treating depression, in which the dosage regimen is established with a method according to the invention or with a computer program product according to the invention. Preferably, for the treatment of ADHD the medicament is selected from methylphenidate, dextroamphetamine, methamphetamine, amphetamine, lisdexamfetamine, and pharmaceutically acceptable salts thereof. Most preferably, the medicament for the treatment of ADHD is methylphenidate or methylphenidate hydrochloride.

In another embodiment, for the treatment of depression the medicament is selected from
- selective serotonin reuptake inhibitors (SSRIs) such as citalopram, escitalopram, fluoxetine, fluvoxamine, paroxetine, sertraline,
- serotonin-norepinephrine reuptake inhibitors (SNRIs) like desvenlafaxine, duloxetine, levomilnacipran, milnacipran, venlafaxine,
- serotonin-norepinephrine-dopamine reuptake inhibitors (SNDRIs) such as toludesvenlafaxine, nefazodone,
- serotonin modulators and stimulators (SMSs) like vilazodone, vortioxetine,
- serotonin antagonist and reuptake inhibitors (SARIs) like nefazodone, trazodone,
- norepinephrine reuptake inhibitors (NRIs) like reboxetine, teniloxazine, viloxazine,
- tricyclic antidepressants (TCAs) such as amitriptyline, amitriptylinoxide, amoxapine, clomipramine, desipramine, dibenzepin, dimetacrine, dosulepin, doxepin, imipramine, lofepramine, melitracen, nitroxazepine, nortriptyline, noxiptiline, pipofezine, protriptyline, trimipramine,
or pharmaceutically acceptable salts of these.

Since all actions taken during the titration process, including adjustments to medication type, dose, frequency, and electronic prescriptions, are documented within the first electronic device and the second electronic device, the method has shown to be more efficient and more secure compared to the titration methods known from the prior art.

## Claims

1. A method for establishing a dosage regimen for a medicament to be administered to a patient suffering from attention deficit hyperactivity disorder (ADHD) or depression comprising a titration phase (t), wherein the titration phase comprises the following steps:
(11) providing baseline data to a first electronic device, wherein the first electronic device is a processing unit, wherein the baseline data includes basic symptoms and weight of the patient to be treated,
(t2) initiating the titration by defining on the first electronic device at least: an initial medicament, an initial dose, and an initial frequency based on the baseline data to obtain initial titration data,
(t3) transferring the initial titration data from the first electronic device to a second electronic device,
(t4) accessing the initial titration data on the second electronic device,
(t5) providing initial symptoms and/or initial side effects to the second electronic device to obtain initial symptoms data,
(t6) transferring the initial symptoms data from the second electronic device to the first electronic device,
(t7) adding the initial symptoms data to the initial titration data to obtain initial symptoms evaluation data.

2. The method according to claim 1, wherein if the initial symptoms evaluation data require an adjustment, after step (t7) the titration phase is repeated with the following steps:
(t2.1) initiating an adjusted titration by defining on the first electronic device at least one of: an adjusted medicament, an adjusted dose, and an adjusted frequency based on the initial symptoms evaluation data to obtain an adjusted titration data,
(t3.1) transferring the adjusted titration data from the first electronic device to the second electronic device,
(t4.1) accessing the adjusted titration data on the second electronic device,
(t5.1) providing symptoms and/or side effects to the second electronic device to obtain adjusted symptoms data,
(t6.1) transferring the adjusted symptoms data from the second electronic device to the first electronic device,
(t7.1) adding the adjusted symptoms data to the initial symptoms evaluation data to obtain adjusted symptoms evaluation data.

3. The method according to claim 2, wherein the steps (t2.1) to (t7.1) are repeated until the adjusted symptoms evaluation data do not require an adjustment.

4. The method according to any one of the preceding claims, wherein step (t2) and/or step (t2.1) comprises setting up a prescription of the medicament.

5. The method according to claim 4, wherein the data submitted to the second electronic device includes the prescription of the medicament.

6. The method according to any one of the preceding claims, comprising a reminder appearing on the second electronic device.

7. The method according to any one of the preceding claims, comprising a visualisation of the initial symptoms evaluation data and/or adjusted symptoms evaluation data obtained in steps (t7) and/or (t7.1).

8. The method according to any one of the preceding claims, comprising a monitoring phase (m) that is commenced after step (t7) or (t7.1).

9. The method according to any one of the preceding claims, wherein the first electronic device is an electronic medical device.

10. The method according to any one of the preceding claims, wherein the second electronic device is an electronic medical device.

11. A computer program product comprising instructions which, when the program is executed by a computer, cause the computer to carry out the steps of the method of any one of claims 1 to 10.

12. A medicament for use in a method of treating attention deficit hyperactivity disorder (ADHD) or depression, wherein the dosage regimen is established with a method according to any one of claims 1 to 10 or with a computer program product according to claim 11.

13. The medicament for use in the method of treating ADHD according to claim 12, wherein the medicament is selected from methylphenidate, dextroamphetamine, methamphetamine, amphetamine, and lisdexamfetamine, or pharmaceutically acceptable salts thereof, preferably methylphenidate, more preferably methylphenidate hydrochloride.

14. The medicament for use in the method of treating depression according to claim 12, wherein the medicament is selected from - selective serotonin reuptake inhibitors (SSRIs) such as citalopram, escitalopram, fluoxetine, fluvoxamine, paroxetine, sertraline, serotonin-norepinephrine reuptake inhibitors (SNRIs) like desvenlafaxine, duloxetine, levomilnacipran, milnacipran, venlafaxine, serotonin-norepinephrine-dopamine reuptake inhibitors (SNDRIs) such as toludesvenlafaxine, nefazodone, serotonin modulators and stimulators (SMSs) like vilazodone, vortioxetine, serotonin antagonist and reuptake inhibitors (SARIs) like nefazodone, trazodone, norepinephrine reuptake inhibitors (NRIs) like reboxetine, teniloxazine, viloxazine, tricyclic antidepressants (TCAs) such as amitriptyline, amitriptylinoxide, amoxapine, clomipramine, desipramine, dibenzepin, dimetacrine, dosulepin, doxepin, imipramine, lofepramine, melitracen, nitroxazepine, nortriptyline, noxiptiline, pipofezine, protriptyline, trimipramine, or pharmaceutically acceptable salts thereof.
